# EUROPÄISCHE PATENTANMELDUNG

(11) **EP 2 158 924 A1**
(43) Veröffentlichungstag der Anmeldung: **03.03.2010**
(21) Anmeldenummer: 08163113.7
(22) Anmeldetag: 27.08.2008
(51) Int. Cl.: A61L 15/26, C08G 18/08, C08G 18/10

(54) **Verfahren zur Herstellung von geformten Polyurethanschaum-Wundauflagen**

(71) Anmelder: Bayer MaterialScience AG, 51368 Leverkusen (DE)
(72) Erfinder: Die Erfindernennung liegt noch nicht vor

(57) **Zusammenfassung**

Die vorliegende Erfindung betrifft ein Verfahren zur Herstellung von geformten Artikeln, wobei auf eine Schaumschicht in einem ersten Bereich bei einer Temperatur von ≥ 0 °C bis ≤ 200 °C ein Druck von ≥ 0 bar bis ≤ 150 bar einwirkt und während der Druckeinwirkung die Schaumschicht in dem ersten Bereich auf > 12,5% bis ≤ 100% ihres ursprünglichen Volumens komprimiert wird; wobei auf eine Schaumschicht in einem zweiten Bereich bei einer Temperatur von ≥ 100 °C bis ≤ 200 °C ein Druck von ≥ 50 bar bis ≤ 150 bar einwirkt und während der Druckeinwirkung die Schaumschicht in dem zweiten Bereich auf > 0% bis ≤ 12,5% ihres ursprünglichen Volumens komprimiert wird; und wobei die Schaumschicht in dem ersten Bereich und dem zweiten Bereich einen Polyurethanschaum umfasst, der erhalten wird, indem eine Zusammensetzung umfassend eine wässrige, anionisch hydrophilierte Polyurethandispersion (I) aufgeschäumt und getrocknet wird. Der Schaum kann mit Ethylenoxid/Propylenoxid-Blockcopolymeren stabilisiert werden. Die Erfindung betrifft weiterhin hiernach erhältliche geformte Artikel sowie deren Verwendung vorzugsweise als Wundauflagen.

## Beschreibung

Die vorliegende Erfindung betrifft ein Verfahren zur Herstellung von geformten Artikeln, wobei eine Schaumschicht in einem bestimmten Bereich zu einem Film verformt wird. Die Schaumschicht umfasst einen Polyurethanschaum, der erhalten wird, indem eine Zusammensetzung umfassend eine wässrige, anionisch hydrophilierte Polyurethandispersion aufgeschäumt und getrocknet wird. Sie betrifft weiterhin gemäß diesem Verfahren hergestellte geformte Artikel sowie deren Verwendung vorzugsweise als Wundauflage.

In der Versorgung von Wunden können Wundauflagen mit einer auf der Wunde liegenden Schaumschicht zum Einsatz kommen. Dieses hat sich als vorteilhaft erwiesen, da über die Aufnahmefähigkeit des Schaums für aus der Wunde austretende Feuchtigkeit ein der Heilung förderliches Klima in der Wunde erreicht werden kann. Bei der Konstruktion von solchen Wundauflagen wird zweckmäßigerweise noch eine Folie oder ein Film vorgesehen, so dass der eigentliche Schaum mit diesem Film verbunden ist. So kann unter Anderem die Wundauflage auf der Haut aufgeklebt werden ("island dressing"). Die mit dem Schaum verbundene Folie oder der Film stellen zusätzlich eine Barriere gegenüber Keimen (zum Beispiel Bakterien) dar. Überdies wird das Austreten von Wundflüssigkeit aus der Wundauflage verhindert. In der Regel wird der Film durch Aufkleben an den Schaum angebracht. Im Übergang zwischen Film und Schaum können sich jedoch Krankheitserreger vermehren, wenn Wundflüssigkeit in diese Bereiche gelangt. Weiterhin können unter Feuchtebelastung solche mit Klebstoff verbundenen Folienbeziehungsweis Film-Schaum-Verbünde versagen, das heisst, das der Schaum sich auf der feuchten Wunde vom Trägermaterial ablöst. Schließlich stellt das Aufkleben eines Films einen weiteren Verfahrensschritt in der Herstellung dar, welcher entsprechende Kosten verursacht.

WO 2007/115696 A1, auf die in vollem Umfang Bezug genommen wird, offenbart ein Verfahren zur Herstellung von Polyurethan-Schäumen für die Wundbehandlung, bei welchem eine Zusammensetzung, enthaltend eine Polyurethan-Dispersion und spezielle Koagulantien, aufgeschäumt und getrocknet wird. Die Polyurethan-Dispersionen können beispielsweise erhalten werden, indem isocyanatfunktionelle Prepolymere aus organischen Polyisocyanaten und polymeren Polyolen mit zahlenmittleren Molekulargewichten von 400 g/mol bis 8000 g/mol und OH-Funktionalitäten von 1,5 bis 6 sowie gegebenenfalls mit hydroxyfunktionellen Verbindungen mit Molekulargewichten von 62 g/mol bis 399 g/mol und gegebenenfalls isocyanatreaktiven, anionischen oder potentiell anionischen und gegebenenfalls nichtionischen Hydrophilierungsmitteln hergestellt werden. Die freien NCO-Gruppen des Prepolymers werden dann ganz oder teilweise gegebenenfalls mit aminofunktionellen Verbindungen mit Molekulargewichten von 32 g/mol bis 400 g/mol sowie mit aminofunktionellen, anionischen oder potentiell anionischen Hydrophilierungsmitteln unter Kettenverlängerung umgesetzt. Die Prepolymere werden vor, während oder nach Schritt der Kettenverlängerung in Wasser dispergiert. Gegebenenfalls enthaltene potentiell ionische Gruppen werden durch teilweise oder vollständige Umsetzung mit einem Neutralisationsmittel in die ionische Form überführt.

GB 2 357 286 A offenbart ein Verfahren zur Herstellung eines geformten Polyurethanschaum-Gegenstands zur Verwendung als oder in einer Wundauflage. Das Verfahren umfasst die Schritte: Bereitstellen eines Leistens mit einer gewünschten dreidimensionalen Form; Auftragen einer wässrigen Schicht über dem Leisten; Auftragen einer Schicht eines isocyanatterminierten Prepolymers über dem Leisten, wobei das Prepolymer mit der wässrigen Schicht auf dem Leisten reagiert unter Ausbildung einer Polyurethanschaumschicht über dem Leisten; sowie Abtragen der Polyurethanschaumschicht von dem Leisten. Vorzugsweise ist der Leisten handförmig und der Gegenstand ein Verbrennungshandschuh. Aus dem erfindungsgemäßen Verfahren erhältliche geformte Polyurethanschaum-Gegenstände werden ebenfalls bereitgestellt. Die Polyurethanschicht ist typischerweise 0,5 bis 10 mm dick und hat eine Dichte von 0,28 g/cm³ und eine Bruchdehnung von mindestens 150%. In diesem Verfahren wird also das Schäumen und Aushärten des Polyurethan-Prepolymers in situ auf einem geeignet geformten Leisten durchgeführt. Aus fertigungstechnischer Sicht ist jedoch nachteilig, dass im letzten Schritt der Herstellung des Gegenstands noch eine chemische Reaktion auftritt, welche Zeit kostet, geeignete Apparaturen braucht und die für chemische Reaktionen nötigen Sicherheitsvorkehrungen erfordert.

WO 2001/00115 A2 offenbart einen geformten Polyurethangegenstand, hergestellt durch das Zusammendrücken eines Polyurethanschaums bei erhöhter Temperatur für eine vorbestimmte Zeit. Es wurde gefunden, dass durch das Zusammendrücken eines Polyurethanschaums in die gewünschte Form, wie sie beispielsweise zum Einführen in Wundkanäle während einer Nasenoperation gebraucht wird, gefolgt vom Aufheizen des Polyurethanschaums auf eine erhöhte Temperatur für eine relativ kurze Zeit, der Polyurethanschaum beim Abkühlen im Wesentlichen seine zusammengedrückte Form beibehält, aber immer noch im Wesentlichen weich und verformbar bleibt. Vorzugsweise sind die Schäume hydrophil und flexibel. Offenbart für das Verfahren werden Polyester- und/oder Polyetherpolyurethane. Die dort offenbarten Schäume werden zylindrisch komprimiert. Es ist nicht bekannt, wie sich die Schäume beim Komprimieren in andere Formen verhalten, also ob beispielsweise bei komplexen Gestaltungen kleine Krümmungsradien korrekt wiedergegeben werden. Weiterhin ist nicht bekannt, wie die Eigenschaften der nur allgemein beschriebenen Schaumtypen durch die thermische Kompression verändern.

Es besteht folglich weiterhin der Bedarf an alternativen geformten Wundauflagen mit einer Schaumschicht und einem die Schaumschicht umgebenden Film oder Folie, wobei hierzwischen keine Lücke auftreten soll. Weiterhin besteht der Bedarf nach einem kostengünstigen Herstellungsverfahren für solche Wundauflagen.

Erfindungsgemäß vorgeschlagen wird daher ein Verfahren zur Herstellung von geformten Artikeln,
wobei auf eine Schaumschicht in einem ersten Bereich bei einer Temperatur von ≥ 0 °C bis ≤ 200 °C ein Druck von ≥ 0 bar bis ≤ 150 bar einwirkt und während der Druckeinwirkung die Schaumschicht in dem ersten Bereich auf > 12,5% bis ≤ 100% ihres ursprünglichen Volumens komprimiert wird;
wobei auf eine Schaumschicht in einem zweiten Bereich bei einer Temperatur von ≥ 100 °C bis ≤ 200 °C ein Druck von ≥ 50 bar bis ≤ 150 bar einwirkt und während der Druckeinwirkung die Schaumschicht in dem zweiten Bereich auf > 0% bis ≤ 12,5% ihres ursprünglichen Volumens komprimiert wird; und
wobei die Schaumschicht in dem ersten Bereich und dem zweiten Bereich einen Polyurethanschaum umfasst, der erhalten wird, indem eine Zusammensetzung umfassend eine wässrige, anionisch hydrophilierte Polyurethandispersion (I) aufgeschäumt und getrocknet wird.

Ein geformter Artikel im Sinne der vorliegenden Erfindung wird durch unterschiedliche Einwirkung von Druck und Temperatur auf verschiedene Bereiche einer Schaumschicht hergestellt. Hierbei behält ein Bereich die Charakteristiken eines Schaums, insbesondere die für die Verwendung als Wundauflage wichtige Aufnahmefähigkeit für Flüssigkeit oder Durchlässigkeit für Wasserdampf. Ein anderer Bereich wird so verändert, dass er zu einem Film oder zu einer Folie verformt wird und somit andere Eigenschaften erhält. Dadurch, dass als Ausgangsmaterial einfach eine Schaumschicht verwendet wurde, sind die beiden Bereiche unmittelbar miteinander verbunden und es treten zwischen den Bereichen keine Lücken auf. Man kann beim geformten Artikel auch von einem integralen Inseldressing sprechen. Mit anderen Worten liegt also der geformte Artikel in Hinsicht auf den ersten und zweiten Bereich der Schaumschicht einstückig vor.

Es ist vorgesehen, dass die Schaumschicht einen Schaum umfasst, welcher aus einer aufgeschäumten Polyurethandispersion erhältlich ist. Diese Schaumschicht wird auf die zu bedeckende Wunde aufgelegt. Vorteilhafterweise weist dieser Schaum eine mikroporöse, zumindest teilweise offenporige Struktur mit miteinander kommunizierenden Zellen auf.

Die Polyurethandispersion (I) umfasst Polyurethane, wobei freie Isocyanatgruppen zumindest teilweise mit anionischen oder potentiell anionischen Hydrophilierungsmitteln umgesetzt wurden. Solche Hydrophilierungsmittel sind Verbindungen, welche gegenüber Isocyanatgruppen reaktive funktionelle Gruppen wie Amino-, Hydroxy- oder Thiolgruppen aufweisen und weiterhin Säure- oder Säureaniongruppen wie Carboxylat-, Sulfonat- oder Phosphonatgruppen.

Nach dem Trocknen der Schaumschicht kann diese vorteilhafterweise als ebene Rollenware bereitgestellt werden. Erfindungsgemäß wirkt auf die Schaumschicht in einem ersten Bereich bei einer Temperatur von ≥ 0 °C bis ≤ 200 °C ein Druck von ≥ 0 bar bis ≤ 150 bar ein und es wird während der Druckeinwirkung die Schaumschicht in dem ersten Bereich auf > 12,5% bis ≤ 100% ihres ursprünglichen Volumens komprimiert. Die Temperatur kann auch in einem Bereich von ≥ 20 °C bis ≤ 100 °C oder von ≥ 23 °C bis ≤ 30 °C liegen. Der Druck kann auch in einem Bereich von ≥ 1 bar bis ≤ 100 bar oder von ≥ 2 bar bis ≤ 50 bar liegen. Die Komprimierung kann auch in einem Bereich von ≥ 25% bis ≤ 90% oder von ≥ 50% bis ≤ 80% des ursprünglichen Volumens liegen. Diese Drücke, Temperaturen und Kompressionen erhalten die Schaumcharakteristiken, so dass dieser Bereich auf eine Wunde aufgelegt werden kann. Welcher Druck, welche Temperatur und Kompression auf ein Volumenelement des ersten Bereichs einwirken, richtet sich unter anderem nach dem Abstand des Volumenelements vom Rand der Aussparung einer Matrize oder aber nach der Form der Matrize selbst, wenn diese Aussparungen aufweist. Die Schaumschicht kann hierbei ebenfalls verformt werden. Beispielsweise kann sie eine halbkugelförmige Gestalt erhalten oder Einbuchtungen erhalten, um Körperteile besser aufnehmen zu können.

Weiterhin ist vorgesehen, dass auf die Schaumschicht in einem zweiten Bereich bei einer Temperatur von ≥ 100 °C bis ≤ 200 °C ein Druck von ≥ 50 bar bis ≤ 150 bar einwirkt und während der Druckeinwirkung die Schaumschicht in dem zweiten Bereich auf > 0% bis ≤ 12,5% ihres ursprünglichen Volumens komprimiert wird. Die Temperatur kann auch in einem Bereich von ≥ 120 °C bis ≤ 190 °C oder von ≥ 150 °C bis ≤ 170 °C liegen. Der Druck kann auch in einem Bereich von ≥ 70 bar bis ≤ 120 bar oder von ≥ 90 bar bis ≤ 110 bar liegen. Die Komprimierung kann auch in einem Bereich von ≥ 5% bis ≤10% oder von ≥ 6% bis ≤ 8% des ursprünglichen Volumens liegen. Bei diesen Bedingungen wird der Schaum zu einem Film oder zu einer Folie komprimiert. Hier kann dann beispielsweise Klebstoff aufgetragen werden, um die Wundauflage auf die Haut aufzukleben.

Weiterhin kann der Film durchsichtig oder durchscheinend erhalten werden, so dass eine Beobachtung des die Wunde umgebenden Gewebes möglich ist. Auf diese Weise kann festgestellt werden, ob sich ein Geschwür weiter ausbreitet oder ob unerwartet viel Exsudat aus der Wunde austritt, ohne dass immer die Wundauflage entfernt werden muss und somit die Gefahr einer Keimbesiedlung der Wunde verringert wird. Zudem erhöht der seltener notwendige Wechsel der Wundauflagen den Patientenkomfort und beschleunigt den Heilungsprozess.

Das Verformen kann in geeigneten Werkzeugen wie zum Beispiel Pressen mit Matrizen und Stempeln durchgeführt werden. In einfachen Fällen kann die Schaumschicht aber auch in einem Kalanderwerkzeug mit einer Krümmung versehen werden, wobei ein Bereich des Schaums nicht von den Kalanderrollen erfasst wird. Bevorzugt werden antihaftbeschichtete Werkzeuge verwendet,
wobei sowohl temporäre Antihaftbeschichtungen beispielsweise durch Aufsprühen von Silikonölen verwendet werden können, als auch entsprechende permanente Beschichtungen wie beispielsweise Teflon- oder Silica-Beschichtungen, wobei im Falle einer Teflonbeschichtung antistatische Teflonbeschichtungen bevorzugt sind. Der Grad der Komprimierung lässt sich im Verformungswerkzeug leicht einstellen, indem ein entsprechender Abstand beispielsweise zwischen Matrize und Stempel oder zwischen Kalanderrollen vorgesehen wird.

Das erfindungsgemäße Verfahren hat den Vorteil, dass aus einer einzigen Schaumlage ein solcher Artikel mit zwei Bereichen hergestellt werden kann. Dieses verringert die Produktionskosten. Weiterhin günstig ist, dass ein Spalt oder Übergang auf der der Wunde zugewandten Seite vermieden wird. Hierdurch vermeidet man die Ansiedlung von Krankheitserregern und kann zusätzlich eine höhere Feuchtebeständigkeit der Wundauflage insgesamt erhalten.

In einer Ausführungsform des erfindungsgemäßen Verfahrens wird das Verformen des zweiten Bereichs für eine Dauer von ≥ 45 Sekunden bis ≤ 90 Sekunden durchgeführt. Die Dauer der Verformung kann auch in einem Bereich von ≥ 50 Sekunden bis ≤ 85 Sekunden oder von ≥ 60 Sekunden bis ≤ 80 Sekunden liegen. Generell ist hierunter die Zeit zu verstehen, in der der zweite Bereich der Schaumschicht unter Einwirkung von Druck und Wärme verformt wird. Mit den erfindungsgemäßen Taktzeiten in der Produktion lassen sich auch im größeren Maßstab erfindungsgemäß verformte Schaumartikel herstellen.

In einer weiteren Ausführungsform des erfindungsgemäßen Verfahrens umfasst die Zusammensetzung, aus der der Polyurethanschaum der Schaumschicht erhalten wird, weiterhin Zusatzstoffe, die ausgewählt sind aus der Gruppe umfassend Fettsäureamide, Sulfosuccinamide, Kohlenwasserstoffsulfonate, Kohlenwasserstoffsulfate, Fettsäuresalze, Alkylpolyglycoside und/oder Ethylenoxid/Propylenoxid-Blockcopolymere.

Solche Zusatzstoffe können als Schaumbildner und/oder Schaumstabilisatoren wirken. In den Fettsäureamiden, Sulfosuccinamiden, Kohlenwasserstoffsulfonaten, Kohlenwasserstoffsulfaten oder Fettsäuresalzen enthält der lipophile Rest bevorzugt ≥ 12 bis ≤ 24 Kohlenstoffatome. Geeignete Alkylpolyglycoside sind beispielsweise durch Umsetzung von längerkettigen Monoalkoholen (≥ 4 bis ≤ 22 C-Atome im Alkylrest) mit Mono-, Di- oder Polysacchariden erhältlich. Weiterhin geeignet sind Alkylbenzosulfonate oder Alkylbenzolsulfate mit ≥ 14 bis ≤ 24 Kohlenstoffatomen im Kohlenwasserstoffrest.

Die Fettsäureamide sind vorzugsweise solche auf Basis von Mono- oder Di-(C₂/C₃-alkanol)aminen. Die Fettsäuresalze können beispielsweise Alkalimetallsalze, Aminsalze oder unsubstituierte Ammoniumsalze sein.

Solche Fettsäurederivate basieren typischerweise auf Fettsäuren wie Laurinsäure, Myristinsäure, Palmitinsäure, Ölsäure, Stearinsäure, Ricinolsäure, Behensäure oder Arachidinsäure, Kokosfettsäure, Talgfettsäure, Sojafettsäure und deren Hydrierungsprodukten.

Beispielhaft verwendbare Schaumstabilisatoren sind Mischungen aus Sulfosuccinamiden und Ammoniumstearaten, wobei diese bevorzugt ≥ 20 Gewichts-% bis ≤ 60 Gewichts-% besonders bevorzugt ≥ 30 Gewichts-% bis ≤ 50 Gewichts-% an Ammoniumstearaten und bevorzugt ≥ 40 Gewichts-%, bis ≤ 80 Gewichts-% besonders bevorzugt ≥ 50 Gewichts-% bis ≤ 70 Gewichts-% an Sulfosuccinamiden enthalten.

Weitere beispielhaft verwendbare Schaumstabilisatoren sind Mischungen aus Fettalkohol-Polyglykosiden und Ammoniumstearaten, wobei diese bevorzugt ≥ 20 Gewichts-% bis ≤ 60 Gewichts-% besonders bevorzugt ≥ 30 Gewichts-% bis ≤ 50 Gewichts-% an Ammoniumstearaten und bevorzugt ≥ 40 Gewichts-%, bis ≤ 80 Gewichts-% besonders bevorzugt ≥ 50 Gewichts-% bis ≤ 70 Gewichts-% an Fettalkohol-Polyglykosiden enthalten.

Bei den Ethylenoxid/Propylenoxid-Blockcopolymere handelt es sich um Additionsprodukte von Ethylenoxid und Propylenoxid an OH- oder NH-funktionelle Startermoleküle.

Als Startermoleküle grundsätzlich geeignet sind unter anderem Wasser, Polyethylenglycole, Polypropylenglycole, Glycerin, Trimethylolpropan, Pentaerythrit, Ethylendiamin, Toluylendiamin, Sorbit, Saccharose und Gemische davon.

Bevorzugt werden als Starter di- oder trifunktionelle Verbindungen der vorstehend genannten Art eingesetzt. Besonders bevorzugt sind Polyethylenglycol oder Polypropylenglycol.

Durch die jeweilige Alkylenoxidmenge und die Anzahl von Ethylenoxid (EO)- und Propylenoxid (PO)-Blöcken lassen sich Blockcopolymere unterschiedlicher Art erhalten.

Grundsätzlich ist es auch möglich, dass die an sich streng blockweise aus Ethylenoxid oder Propylenoxid aufgebauten Copolymere auch einzelne Mischblöcke aus EO und PO aufweisen.

Solche Mischblöcke werden erhalten, wenn in die Polyadditionsreaktion Mischungen aus EO und PO eingesetzt werden, so dass bezogen auf diesen Block eine statistische Verteilung von EO und PO in diesem Block resultiert.

Bevorzugt weisen die erfindungsgemäß eingesetzten EO/PO-Blockcopolymere Gehalte an Ethylenoxideinheiten von ≥ 5 Gewichts-%, besonders bevorzugt ≥ 20 Gewichts-% und ganz besonders bevorzugt ≥ 40 Gewichts-% bezogen auf die Summe der im Copolymer vorliegenden Ethylenoxid- und Propylenoxideinheiten auf.

Bevorzugt weisen die erfindungsgemäß eingesetzten EO/PO-Blockcopolymere Gehalte an Ethylenoxideinheiten von ≤ 95 Gewichts-%, besonders bevorzugt ≤ 90 Gewichts-% und ganz besonders bevorzugt ≤ 85 Gewichts-% bezogen auf die Summe der im Copolymer vorliegenden Ethylenoxid- und Propylenoxideinheiten auf.

Bevorzugt weisen die erfindungsgemäß eingesetzten EO/PO-Blockcopolymere zahlenmittlere Molekulargewichte von ≥ 1000 g/mol, besonders bevorzugt ≥ 2000 g/mol, ganz besonders bevorzugt ≥ 5000 g/mol auf.

Bevorzugt weisen die erfindungsgemäß eingesetzten EO/PO-Blockcopolymere zahlenmittlere Molekulargewichte von ≤ 10000 g/mol, besonders bevorzugt ≤ 9500 g/mol, ganz besonders bevorzugt ≤ 9000 g/mol auf.

Vorteilhaft an der Verwendung der EO/PO-Blockcopolymere ist, dass der erhaltene Schaum eine geringere Hydrophobie als bei Verwendung anderer Stabilisatoren aufweist. Hierdurch kann das Aufnahmeverhalten für Flüssigkeiten günstig beeinflusst werden. Zudem werden bei der Verwendung der EO/PO-Blockcopolymere im Gegensatz zu anderen Stabilisatoren nichtzytotoxische Schäume erhalten.

Es ist möglich, dass die Ethylenoxid/Propylenoxid-Blockcopolymere eine Struktur gemäß der allgemeinen Formel (1) aufweisen: 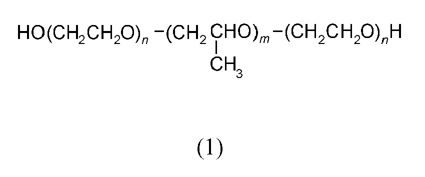 wobei der Wert für n in einem Bereich von ≥ 2 bis ≤ 200 liegt und der Wert für m in einem Bereich von ≥ 10 bis ≤ 60 liegt.

Besonders bevorzugt sind EO/PO-Blockcopolymere der vorstehend genannten Art, wobei diese einen hydrophilic-lipophilic-balance (HLB)-Wert von ≥ 4, besonders bevorzugt von ≥ 8 und ganz besonders bevorzugt von ≥ 14 besitzen. Der HLB-Wert errechnet sich nach der Formel HLB = 20 · Mh/M, wobei Mh die zahlenmittlere Molmasse des hydrophilen aus Ethylenoxid gebildeten Anteils des Moleküls und M die zahlenmittlere Molmasse des gesamten Moleküls ist (Griffin, W.C.: Classification of surface active agents by HLB, J. Soc. Cosmet. Chem. 1, 1949). Der HLB-Wert liegt jedoch bei ≤ 19, bevorzugt bei ≤ 18.

In einer Ausführungsform des erfindungsgemäßen Verfahrens ist die wässrige, anionisch hydrophilierte Polyurethandispersion (I) erhältlich, indem
A) isocyanatfunktionelle Prepolymere bereitgestellt werden, welche erhältlich sind aus einer Reaktionsmischung umfassend
   A1) organische Polyisocyanate und
   A2) polymere Polyole mit zahlenmittleren Molekulargewichten von ≥ 400 g/mol bis ≤ 8000 g/mol und OH-Funktionalitäten von ≥ 1,5 bis ≤ 6
   und wobei anschließend
B) die freien NCO-Gruppen der Prepolymere ganz oder teilweise mit
   B1) aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden, wobei weiterhin in der Reaktionsmischung vorliegende potentiell ionische Gruppen durch teilweise oder vollständige Umsetzung mit einem Neutralisationsmittel in die ionische Form überführt werden.

Bevorzugte wässrige, anionische Polyurethan-Dispersionen (I) haben einen niedrigen Grad an hydrophilen anionischen Gruppen, bevorzugt von ≥ 0,1 bis ≤ 15 Milliequivalenten pro 100 g Festharz.

Um eine gute Sedimentationsstabilität zu erreichen, liegt die zahlenmittlere Teilchengröße der speziellen Polyurethan-Dispersionen bevorzugt bei ≤ 750 nm, besonders bevorzugt bei ≤ 500 nm, bestimmt mittels Laserkorrelations-Spektroskopie.

Das Verhältnis von NCO-Gruppen der Verbindungen aus Komponente A1) zu NCO-reaktiven Gruppen wie Amino-, Hydroxy- oder Thiolgruppen der Verbindungen der Komponenten A2) bis A4) beträgt bei der Herstellung des NCO-funktionellen Prepolymers ≥ 1,05 bis ≤ 3,5, bevorzugt ≥ 1,2 bis ≤ 3,0 besonders bevorzugt ≥ 1,3 bis ≤ 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers ≥ 40% bis ≤ 150%, bevorzugt zwischen ≥ 50% und ≤ 125%, besonders bevorzugt zwischen ≥ 60% und ≤ 120% beträgt.

Geeignete Polyisocyanate der Komponente A1) sind aromatische, araliphatische, aliphatische oder cycloaliphatische Polyisocyanate mit einer NCO-Funktionalität von ≥ 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat (TDI), 1,5-Naphthylendiisocyanat, 2,2'- und/oder 2,4'- und/oder 4,4'-Diphenylmethandiisocyanat (MDI), 1,3- und/oder 1,4-Bis-(2-isocyanato-prop-2-yl)-benzol (TMXDI), 1,3-Bis- (isocyanatomethyl)benzol (XDI), sowie Alkyl-2,6-diisocyanatohexanoate (Lysindiisocyanate) mit C₁- bis C₈-Alkylgruppen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül wie zum Beispiel 4-Isocyanatomethyl-1,8-octandiisocyanat (Nonantriisocyanat) oder Triphenylmethan- 4,4',4"-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von ≥ 2 bis ≤ 4, bevorzugt ≥ 2 bis ≤ 2,6 und besonders bevorzugt ≥ 2 bis ≤ 2,4.

Besonders bevorzugt werden in A1) 1,6-Hexamethylendiisocyanat, Isophorondiisocyanat, die isomeren Bis(4,4'-isocyanatocyclohexyl)methane sowie deren Mischungen eingesetzt.

In A2) werden polymere Polyole mit einem zahlenmittleren Molekulargewicht Mn von ≥ 400 g/mol bis ≤ 8000 g/mol, bevorzugt von ≥ 400 g/mol bis ≤ 6000 g/mol und besonders bevorzugt von ≥ 600 g/mol bis ≤ 3000 g/mol eingesetzt. Diese weisen bevorzugt eine OH-Funktionalität von ≥ 1,5 bis ≤ 6, besonders bevorzugt von ≥ 1,8 bis ≤ 3, ganz besonders bevorzugt von ≥ 1,9 bis ≤ 2,1 auf.

Solche polymeren Polyole sind beispielsweise Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Solche Polyesterpolyole sind Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Triemthylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2- Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols ≥ 2 ist, können zusätzlich auch Monocarbonsäuren wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) Hydroxylgruppen aufweisende Polycarbonate, bevorzugt Polycarbonatdiole, mit zahlenmittleren Molekulargewichten Mn von ≥ 400 g/mol bis ≤ 8000 g/mol, bevorzugt ≥ 600 g/mol bis ≤ 3000 g/mol eingesetzt werden. Diese sind durch Reaktion von Kohlensäurederivaten wie Diphenylcarbonat, Dimethylcarbonat oder Phosgen mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol ≥ 40 Gewichts-% bis ≤ 100 Gewichts-% Hexandiol, bevorzugt 1,6-Hexandiol und/oder Hexandiolderivate. Solche Hexandiolderivate basieren auf Hexandiol und weisen neben endständigen OH-Gruppen auch Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Die Hydroxylgruppen aufweisenden Polycarbonate sind bevorzugt linear gebaut.

Ebenfalls können in A2) Polyetherpolyole eingesetzt werden.

Geeignet sind beispielsweise Polytetramethylenglykolpolyether, wie sie durch Polymerisation von Tetrahydrofuran mittels kationischer Ringöffhung erhältlich sind.

Ebenfalls geeignete Polyetherpolyole sind die Additionsprodukte von Styroloxid, Ethylenoxid, Propylenoxid, Butylenoxid und/oder Epichlorhydrin an di- oder polyfunktionelle Startermoleküle. Polyetherpolyole, basierend auf der zumindest anteiligen Addition von Ethylenoxid an di- oder polyfunktionelle Startermoleküle, können auch als Komponente A4) eingesetzt werden (nichtionische Hydrophilierungsmittel).

Als geeignete Startermoleküle eingesetzt werden können beispielsweise Wasser, Butyldiglykol, Glycerin, Diethylenglykol, Trimethyolpropan, Propylenglykol, Sorbit, Ethylendiamin, Triethanolamin oder 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Ausführungsformen der Polyurethan-Dispersionen (I) enthalten als Komponente A2) eine Mischung aus Polycarbonatpolyolen und Polytetramethylenglykolpolyolen, wobei in dieser Mischung der Anteil an Polycarbonatpolyolen in der Mischung ≥ 20 Gewichts-% bis ≤ 80 Gewichts-% und der Anteil an Polytetramethylenglykolpolyolen ≥ 20 Gewichts-% bis ≤ 80 Gewichts-% beträgt. Bevorzugt ist ein Anteil von ≥ 30 Gewichts-% bis ≤ 75 Gewichts-% an Polytetramethylenglykolpolyolen und ein Anteil von ≥ 25 Gewichts-% bis ≤ 70 Gewichts-% an Polycarbonatpolyolen. Besonders bevorzugt ist ein Anteil von ≥ 35 Gewichts-% bis ≤ 70 Gewichts-% an Polytetramethylenglykolpolyolen und ein Anteil von ≥ 30 Gewichts-% bis ≤ 65 Gewichts-% an Polycarbonatpolyolen, jeweils mit der Maßgabe, dass die Summe der Gewichtsprozente der Polycarbonatpolyole und Polytetramethylenglykolpolyole ≤ 100 Gewichts-% ergibt und der Anteil der Summe der Polycarbonatpolyole und Polytetramethylenglykolpolyetherpolyole an der Komponente A2) ≥ 50 Gewichts-%, bevorzugt ≥ 60 Gewichts-% und besonders bevorzugt ≥ 70 Gewichts-% beträgt.

Unter isocyanatreaktiven anionischen oder potentiell anionischen Hydrophilierungsmitteln der Komponente B1) werden sämtliche Verbindungen verstanden, die mindestens eine isocyanatreaktive Gruppe wie eine Amino-, Hydroxy- oder Thiolgruppe aufweisen, sowie mindestens eine Funktionalität wie zum Beispiel -COO⁻M⁺, -SO₃⁻M⁺, -PO(O⁻M⁺)₂ mit M⁺ beispielsweise gleich Metallkation, H⁺, NH₄⁺, NHR₃⁺, wobei R jeweils ein C₁-C₁₂-Alkylrest, C₅-C₆-Cycloalkylrest und/oder ein C₂-C₄-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann.

Vorzugsweise sind die isocyanatreaktiven anionischen oder potentiell anionischen Hydrophilierungsmittel isocyanatreaktive aminofunktionelle anionische oder potentiell anionische Hydrophilierungsmittel.

Geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen oder potentiell anionischen Hydrophilierungsmittel sind N-(2-Aminoethyl)-β-alanin, 2-(2-Amino-ethylamino)-ethansulfonsäure, Ethylendiaminpropyl- oder -butylsulfonsäure, 1,2- oder 1,3-Propylendiamin-βethylsulfonsäure, Glycin, Alanin, Taurin, Lysin, 3,5-Diaminobenzoesäure und das Additionsprodukt von IPDA und Acrylsäure (EP- A 0 916 647, Beispiel 1). Weiterhin kann Cyclohexylaminopropansulfonsäure (CAPS) aus WO-A 01/88006 als anionisches oder potentiell anionisches Hydrophilierungsmittel verwendet werden.

Bevorzugte anionische oder potentiell anionische Hydrophilierungsmittel der Komponente B1) sind solche der vorstehend genannten Art, die über Carboxylat- beziehungsweise Carobonsäuregruppen und/oder Sulfonatgruppen verfügen, wie die Salze von N-(2-Aminoethyl)-β-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen beziehungsweise potentiell anionischen Hydrophilierungsmitteln und nichtionischen Hydrophilierungsmitteln verwendet werden.

In einer weiteren Ausführungsform des erfindungsgemäßen Verfahrens umfasst die Reaktionsmischung in Schritt A) weiterhin:
A3) hydroxyfunktionelle Verbindungen mit Molekulargewichten von ≥ 62 g/mol bis ≤ 399 g/mol

Die Verbindungen der Komponente A3) besitzen Molekulargewichte von ≥ 62 g/mol bis ≤ 399 g/mol.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4- Butandiol, 1,3-Butylenglykol, Cyclohexandiol, 1 ,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-εhydroxycapronsäureester, ω-Hydroxyhexyl-γ-hydroxybuttersäureester, Adipinsäure-(β-hydroxyethyl)ester oder Terephthalsäurebis(β-hydroxyethyl)-ester.

Ferner können in A3) auch monofunktionelle, isocyanatreaktive, Hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobutylether, Diethylenglykolmonobutylether, Propylenglykolmonomethylether, Dipropylenglykolmonomethylether, Tripropylenglykolmonomethylether, Dipropylenglykolmonopropylether, Propylenglykolmonobutylether, Dipropylenglykolmonobutylether, Tripropylenglykolmonobutylether, 2- Ethylhexanol, 1-Octanol, 1-Dodecanol, 1-Hexadecanol.

Bevorzugte Verbindungen der Komponente A3) sind 1,6-Hexandiol, 1,4-Butandiol, Neopentylglykol und Trimethylolpropan.

In einer weiteren Ausführungsform des erfindungsgemäßen Verfahrens umfasst die Reaktionsmischung in Schritt A) weiterhin:
A4) isocyanatreaktive, anionische oder potentiell anionische und gegebenenfalls nichtionische Hydrophilierungsmittel

Unter anionisch beziehungsweise potentiell anionisch hydrophilierenden Verbindungen der Komponente A4) werden sämtliche Verbindungen verstanden, die mindestens eine isocyanatreaktive Gruppe wie eine Amino-, Hydroxyl- oder Thiolgruppe aufweisen sowie mindestens eine Funktionalität wie zum Beispiel -COO⁻M⁺, -SO₃⁻M⁺, -PO(O⁻M⁺)₂ mit M⁺ beispielsweise gleich Metallkation, H⁺, NH₄⁺, NHR₃⁺, wobei R jeweils ein C₁-C₁₂-Alkylrest, C₅-C₆-Cycloalkylrest und/oder ein C₂-C₄-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind zum Beispiel Mono- und Dihydroxycarbonsäuren, Mono- und Dihydroxysulfonsäuren sowie Mono- und Dihydroxyphosphonsäuren und ihre Salze. Beispiele solcher anionischen beziehungsweise potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5 - 9, Formel I-III beschrieben ist. Bevorzugte anionische oder potentiell anionische Hydrophilierungsmittel der Komponente A4) sind solche der vorstehend genannten Art, die über Carboxylat- beziehungsweise Carbonsäuregruppen und/oder Sulfonatgruppen verfügen.

Besonders bevorzugte anionische oder potentiell anionische Hydrophilierungsmittel sind solche, die Carboxylat- beziehungsweise Carbonsäuregruppen als ionische oder potentiell ionische Gruppen enthalten wie Dimethylolpropionsäure, Dimethylolbuttersäue und Hydroxypivalinsäure und/oder deren Salze.

Geeignete nichtionisch hydrophilierende Verbindungen der Komponente A4) sind zum Beispiel Polyoxyalkylenether, die mindestens eine Hydroxy- oder Aminogruppe, bevorzugt mindestens eine Hydroxygruppe enthalten. Beispiele hierfür sind die monohydroxyfunktionellen, im statistischen Mittel ≥ 5 bis ≤ 70, bevorzugt ≥ 7 bis ≤ 55 Ethylenoxideinheiten pro Molekül aufweisenden Polyalkylenoxidpolyetheralkohole, wie durch Alkoxylierung geeigneter Startermoleküle zugänglich sind. Diese sind entweder reine Polyethylenoxidether oder gemischte Polyalkylenoxidether, wobei sie ≥ 30 mol-%, bevorzugt ≥ 40 mol-% bezogen auf alle enthaltenen Alkylenoxideinheiten an Ethylenoxideinheiten enthalten.

Bevorzugte Polyethylenoxidether der vorstehend genannten Art sind monofunktionelle gemischte Polyalkylenoxidpolyether, die ≥ 40 mol-% bis ≤ 100 mol-% Ethylenoxid- und ≥ 0 mol-% bis ≤ 60 mol-% Propylenoxideinheiten aufweisen.

Bevorzugte nichtionisch hydrophilierende Verbindungen der Komponente A4) sind solche der vorstehend genannten Art, wobei es sich um Block(co)polymere handelt, die durch blockweise Addition von Alkylenoxiden an geeignete Starter hergestellt werden.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

In einer weiteren Ausführungsform des erfindungsgemäßen Verfahrens werden in Schritt B) die freien NCO-Gruppen der Prepolymere weiterhin ganz oder teilweise umgesetzt mit
B2) aminofunktionelle Verbindungen mit Molekulargewichten von ≥ 32 g/mol bis ≤ 400 g/mol

Als Komponente B2) können Di- oder Polyamine wie 1,2-Ethylendiamin, 1,2- und 1,3-Diaminopropan, 1,4-Diaminobutan, 1,6-Diaminohexan, Isophorondiamin, Isomerengemische von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin sowie Hydraziden wie Adipinsäuredihydrazid.

Darüber hinaus können als Komponente B2) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-Methylaminobutan, Alkanolamine wie N-Aminoethylethanolamin, Ethanolamin, 3-Aminopropanol, Neopentanolamin.

Ferner können als Komponente B2) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin oder geeignete substituierte Derivate davon, Amidamine aus diprimären Aminen und Monocarbonsäuren, Monoketime von diprimären Aminen, primär/tertiäre Amine, wie N,N-Dimethylaminopropylamin. Bevorzugte Verbindungen der Komponente B2) sind 1,2-Ethylendiamin, 1,4-Diaminobutan und Isophorondiamin.

In einer weiteren Ausführungsform des erfindungsgemäßen Verfahrens ist bei der Herstellung der wässrigen, anionisch hydrophilierten Polyurethan-Dispersionen (I) die Komponente A1) ausgewählt aus der Gruppe umfassend 1,6-Hexamethylendiisocyanat, Isophorondiisocyanat und/oder die isomeren Bis-(4,4'-isocyanatocyclohexyl)methane und wobei weiterhin die Komponente A2) eine Mischung aus Polycarbonatpolyolen und Polytetramethylenglykolpolyolen umfasst, wobei der Anteil der Summe der Polycarbonatpolyole und der Polytetramethylenglykolpolyetherpolyole an der Komponente A2) ≥ 70 Gewichts-% bis ≤ 100 Gewichts-% beträgt.

Neben den Polyurethan-Dispersionen (I) und den Zusatzstoffen können auch weitere Hilfsstoffe verwendet werden.

Beispiele für solche Hilfsstoffe sind Verdicker beziehungsweise Thixotropiermittel, Antioxidantien, Lichtschutzmittel, Emulgatoren, Weichmacher, Pigmente, Füllstoffe und/oder Verlaufshilfsmittel.

Als Verdicker können handelsübliche Verdicker eingesetzt werden, wie Dextrin-, Stärke- oder Cellulosederivate wie Celluloseether oder Hydroxyethylcellulose, Polysaccharidderivate wie Gummi arabicum oder Guar, organische vollsynthetische Verdicker auf Basis von Polyacrylsäuren, Polyvinylpyrrolidonen, Poly(meth)acrylverbindungen oder Polyurethanen (assoziative Verdicker) sowie anorganische Verdicker wie Bentonite oder Kieselsäuren.

Grundsätzlich können die erfindungsgemäßen Zusammensetzungen auch Vernetzer wie unblockierte Polyisocyanate, Amid- und Amin-Formaldehydharze, Phenolharze, Aldehyd- und Ketonharze, wie zum Beispiel Phenol-Formaldehydharze, Resole, Furanharze, Harnstoffharze, Carbamidsäureesterharze, Triazinharze, Melaminharze, Benzoguanaminharze, Cyanamidharze oder Anilinharze enthalten.

In einer beispielhaften Rezeptur zur Herstellung der Polyurethan-Dispersionen werden die Komponenten A1) bis A4) und B1) bis B2) in den folgenden Mengen eingesetzt, wobei sich die Einzelmengen stets zu ≤ 100 Gewichts-% addieren:
≥ 5 Gewichts-% bis ≤ 40 Gewichts-% der Komponente A1);
≥ 55 Gewichts-% bis ≤ 90 Gewichts-% der Komponente A2);
≥ 0,5 Gewichts-% bis ≤ 20 Gewichts-% Summe der Komponenten A3) und B2);
≥ 0,1 Gewichts-% bis ≤ 25 Gewichts-% Summe der Komponenten A4) und B1), wobei bezogen auf die Gesamtmengen der Komponenten A1) bis A4) und B1) bis B2) ≥ 0,1 Gewichts-% bis ≤ 5 Gewichts-% an anionischen oder potentiell anionischen Hydrophilierungsmitteln aus A4) und/oder B1) verwendet werden.

In einer weiteren beispielhaften Rezeptur zur Herstellung der Polyurethan-Dispersionen werden die Komponenten A1) bis A4) und B1) bis B2) in den folgenden Mengen eingesetzt, wobei sich die Einzelmengen stets zu ≤ 100 Gewichts-% aufaddieren:
≥ 5 Gewichts-% bis ≤ 35 Gewichts-% der Komponente A1);
≥ 60 Gewichts-% bis ≤ 90 Gewichts-% der Komponente A2);
≥ 0,5 Gewichts-% bis ≤ 15 Gewichts-% Summe der Komponenten A3) und B2);
≥ 0,1 Gewichts-% bis ≤ 15 Gewichts-% Summe der Komponenten A4) und B1), wobei bezogen auf die Gesamtmengen der Komponenten A1) bis A4) und B1) bis B2) ≥ 0,2 Gewichts-% bis ≤ 4 Gewichts-% an anionischen bzw. potentiell anionischen Hydrophilierungsmitteln aus A4) und/oder B1) verwendet werden.

In einer ganz besonders bevorzugten Rezeptur zur Herstellung der Polyurethan-Dispersionen werden die Komponenten A1) bis A4) und B1) bis B2) in den folgenden Mengen eingesetzt, wobei sich die Einzelmengen stets zu ≤ 100 Gewichts-% aufaddieren:
≥ 10 Gewichts-% bis ≤ 30 Gewichts-% der Komponente A1);
≥ 65 Gewichts-% bis ≤ 85 Gewichts-% der Komponente A2);
≥ 0,5 Gewichts-% bis ≤ 14 Gewichts-% Summe der Komponenten A3) und B2);
≥ 0,1 Gewichts-% bis ≤ 13,5 Gewichts-% Summe der Komponenten A4) und B1), wobei bezogen auf die Gesamtmengen der Komponenten A1) bis A4) und B1) bis B2) ≥ 0,5 Gewichts-% bis ≤ 3,0 Gewichts-% an anionischen oder potentiell anionischen Hydrophilierungsmitteln aus A4) und/oder B1) verwendet werden.

Die Herstellung der anionisch hydrophilierten Polyurethan-Dispersionen (I) kann in einer oder mehreren Stufe/-n in homogener oder bei mehrstufiger Umsetzung, teilweise in disperser Phase durchgeführt werden. Nach vollständig oder teilweise durchgeführter Polyaddition aus A1 ) bis A4) erfolgt ein Dispergier-, Emulgier- oder Lösungsschritt. Im Anschluss erfolgt gegebenenfalls eine weitere Polyaddition oder Modifikation in disperser Phase.

Dabei können Verfahren wie beispielsweise Prepolymer- Mischverfahren, Acetonverfahren oder Schmelzdispergierverfahren verwendet werden. Bevorzugt wird das Aceton-Verfahren verwendet.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von ≥ 50 °C bis ≤ 120 °C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton oder 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von N-Methylpyrrolidon, N-Ethylpyrrolidon in der Dispersion verbleiben. Bevorzugt werden aber außer den üblichen aliphatischen, ketofunktionellen Lösemitteln keine anderen Lösungsmittel verwendet.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanatgruppen zu mit isocyanatreaktiven Gruppen beispielsweise ≥ 1,05 bis ≤ 3,5, bevorzugt ≥ 1,2 bis ≤ 3,0 und besonders bevorzugt ≥ 1,3 bis ≤ 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, zum Beispiel Trialkylamine mit ≥ 1 bis ≤ 12, bevorzugt ≥ 1 bis ≤ 6 C-Atomen, besonders bevorzugt ≥ 2 bis ≤ 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-Methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt zwischen ≥ 50 mol-% und ≤ 125 mol%, bevorzugt zwischen ≥ 70 mol-% und ≤ 100 mol% der Stoffmenge der zu neutralisierenden Säuregruppen. Die Neutralisation kann auch gleichzeitig mit der Dispergierung erfolgen, indem das Dispergierwasser bereits das Neutralisationsmittel enthält.

Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung in Stufe B) werden NH₂- und/oder NH-funktionelle Komponenten mit den noch verbliebenen Isocyanatgruppen des Prepolymers teilweise oder vollständig umgesetzt. Bevorzugt wird die Kettenverlängerung vor der Dispergierung in Wasser durchgeführt.

Zur Kettenterminierung werden üblicherweise Amine B2) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B1) mit NH₂- oder NH-Gruppen eingesetzt, erfolgt die Kettenverlängerung der Prepolymere bevorzugt vor der Dispergierung.

Die aminischen Komponenten B1) und B2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt ≥ 70 Gewichts-% bis ≤ 95 Gewichts-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie zum Beispiel starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen (I) beträgt typischerweise ≤ 1,0 Gew.-%, bevorzugt ≤ 0,5 Gew.-%, bezogen auf die gesamte Dispersion.

Der pH-Wert der erfindungsgemäßen Polyurethan-Dispersionen (I) beträgt typischerweise ≤ 9,0, bevorzugt ≤ 8,5, besonders bevorzugt weniger als ≤ 8,0 und liegt ganz besonders bevorzugt bei ≥ 6,0 bis ≤ 7,5.

Der Feststoffgehalt der Polyurethan-Dispersionen (I) beträgt bevorzugt ≥ 40 Gewichts-% bis ≤ 70 Gewichts-%, besonders bevorzugt ≥ 50 Gewichts-% bis ≤ 65 Gewichts-%, ganz besonders bevorzugt ≥ 55 Gewichts-% bis ≤ 65 Gewichts-% und insbesondere ≥ 60 Gewichts-% bis ≤ 65 Gewichts-%.

Beispiele für erfindungsgemäße Zusammensetzungen werden nachfolgend aufgeführt, wobei die Summe der Angaben in Gewichts-% einen Wert von ≤ 100 Gewichts-% einnimmt. Diese Zusammensetzungen umfassen, bezogen auf die Trockensubstanz, typischerweise ≥ 80 Gewichtsteile bis ≤ 99,5 Gewichtsteile der Dispersion (I), ≥ 0 Gewichtsteile bis ≤ 10 Gewichtsteile Schaumhilfsmittel, ≥ 0 Gewichtsteile bis ≤ 10 Gewichtsteile Vernetzer und ≥ 0 Gewichtsteile bis ≤ 10 Gewichtsteile Verdicker.

Bevorzugt umfassen diese erfindungsgemäßen Zusammensetzungen, bezogen auf die Trockensubstanz, ≥ 85 Gewichtsteile bis ≤ 97 Gewichtsteile der Dispersion (I), ≥ 0,5 Gewichtsteile bis ≤ 7 Gewichtsteile Schaumhilfsmittel, ≥ 0 Gewichtsteile bis ≤ 5 Gewichtsteile Vernetzer und ≥ 0 Gewichtsteile bis ≤ 5 Gewichtsteile Verdicker.

Besonders bevorzugt umfassen diese erfindungsgemäßen Zusammensetzungen, bezogen auf die Trockensubstanz, ≥ 89 Gewichtsteile bis ≤ 97 Gewichtsteile der Dispersion (I), ≥ 0,5 Gewichtsteile bis ≤ 6 Gewichtsteile Schaumhilfsmittel, ≥ 0 Gewichtsteile bis ≤ 4 Gewichtsteile Vernetzer und ≥ 0 Gewichtsteile bis ≤ 4 Gewichtsteile Verdicker.

Beispiele für erfindungsgemäße Zusammensetzungen, welche Ethylenoxid/Propylenoxid-Blockcopolymere als Schaumstabilisatoren umfassen, werden nachfolgend aufgeführt. Diese Zusammensetzungen umfasse, bezogen auf Trockensubstanz, ≥ 80 Gewichtsteile bis ≤ 99,9 Gewichtsteile der Dispersion (I) und ≥ 0,1 Gewichtsteile bis ≤ 20 Gewichtsteile der Ethylenoxid/Propylenoxid-Blockcopolymere. Bevorzugt umfassen die Zusammensetzungen, bezogen auf Trockensubstanz, ≥ 85 Gewichtsteile bis ≤ 99,5 Gewichtsteile der Dispersion (I) und 0,5 bis 15 Gewichtsteile der Ethylenoxid/Propylenoxid-Blockcopolymere. Besonders bevorzugt sind hierbei ≥ 90 Gewichtsteile bis ≤ 99 Gewichtsteile der Dispersion (I) und ≥ 1 Gewichtsteile bis ≤ 10 Gewichtsteile der Ethylenoxid/Propylenoxid-Blockcopolymere und ganz besonders bevorzugt ≥ 94 Gewichtsteile bis ≤ 99 Gewichtsteile der Dispersion (I) und ≥ 1 bis ≤ 6 Gewichtsteile der Ethylenoxid/Propylenoxid-Blockcopolymere.

Im Sinne der vorliegenden Erfindung bedeutet die Angabe "Gewichtsteile" einen relativen Anteil, jedoch nicht im Sinne der Angabe von Gewichts-%. Folglich kann die zahlenmäßige Summe der Gewichtsanteile auch Werte über 100 annehmen.

Neben den genannten Komponenten können in den erfindungsgemäßen Zusammensetzungen auch weitere wässrige Bindemittel eingesetzt werden. Solche wässrigen Bindemittel können zum Beispiel aus Polyester-, Polyacrylat-, Polyepoxid- oder anderen Polyurethanpolymeren aufgebaut sein. Auch die Kombination mit strahlenhärtbaren Bindemitteln, wie sie beispielsweise in der EP-A-0 753 531 beschrieben sind, ist möglich. Ferner können auch andere anionische oder nichtionische Dispersionen, wie Polyvinylacetat-, Polyethylen-, Polystyrol-, Polybutadien-, Polyvinylchlorid-, Polyacrylat- und Copolymerisat-Dispersionen eingesetzt werden.

Das Aufschäumen im erfindungsgemäßen Verfahren geschieht durch mechanisches Rühren der Zusammensetzung bei hohen Drehzahlen, durch Schütteln oder durch Entspannung eines Treibgases.

Das mechanische Aufschäumen kann mit beliebigen mechanischen Rühr-, Misch- und Dispergiertechniken erfolgen. In der Regel wird hierbei Luft eingetragen, aber auch Stickstoff und andere Gase können hierfür benutzt werden.

Der erhaltene Schaum wird beim Aufschäumen oder unmittelbar danach auf ein Substrat aufgetragen oder in eine Form gegeben und getrocknet. Als Substrate geeignet sind insbesondere Papiere oder Folien, die ein einfaches Ablösen der Wundauflage vor ihrem Einsatz zur Abdeckung einer verletzten Stelle ermöglichen.

Der Auftrag kann beispielsweise durch Gießen oder Rakeln erfolgen, aber auch andere an sich bekannte Techniken sind möglich. Ein mehrschichtiger Auftrag mit zwischengeschalteten Trocknungsschritten ist grundsätzlich auch möglich.

Eine befriedigende Trocknungsgeschwindigkeit der Schäume wird bereits bei 20 °C beobachtet, so dass eine Trocknung auf verletztem menschlichen oder tierischen Gewebe problemlos möglich ist. Für eine schnellere Trocknung und Fixierung der Schäume werden jedoch bevorzugt Temperaturen oberhalb von 30 °C benutzt. Bei der Trocknung sollten jedoch Temperaturen von 200 °C, bevorzugt 150 °C, besonders bevorzugt 130 °C nicht überschritten werden, da es anderenfalls zu unerwünschter Vergilbung der Schäume kommen kann. Möglich ist auch eine zwei- oder mehrstufige Trocknung.

Die Trocknung erfolgt in der Regel unter Verwendung von an sich bekannten Heiz- und Trockenapparaten, wie (Umluft-) Trockenschränken, Heißluft oder IR-Strahlern. Auch die Trocknung durch Führen des beschichteten Substrates über geheizte Oberflächen, zum Beispiel Walzen, ist möglich.

Der Auftrag sowie die Trocknung können jeweils diskontinuierlich oder kontinuierlich durchgeführt werden, bevorzugt ist jedoch ein gänzlich kontinuierliches Verfahren.

Die Polyurethan-Schäume können vor ihrer Trocknung typischerweise Schaumdichten von ≥ 50 g/Liter bis ≤ 800 g/Liter, bevorzugt ≥ 100 g/Liter bis ≤ 500 g/Liter, besonders bevorzugt ≥ 100 g/Liter bis ≤ 250 g/Liter (Masse aller Einsatzstoffe [in g] bezogen auf das Schaumvolumen von einem Liter).

Nach ihrer Trocknung können die Schäume eine mikroporöse, zumindest teilweise offenporige Struktur mit miteinander kommunizierenden Zellen aufweisen. Die Dichte der getrockneten Schäume liegt dabei typischerweise unterhalb von 0,4 g/cm³, bevorzugt ist sie geringer als 0,35 g/cm³, besonders bevorzugt ≥ 0,01 g/cm³ bis ≤ 0,3 g/cm³ und liegt ganz besonders bevorzugt bei ≥ 0,1 g/cm³ bis ≤ 0,3 g/cm³.

Gegenstand der Erfindung ist weiterhin ein geformter Artikel, erhältlich durch ein erfindungsgemäßes Verfahren, mit einem Schaumbereich und einem komprimierten Bereich, wobei der komprimierte Bereich aus der Kompression einer Schaumschicht erhalten wird und wobei diese Schaumschicht sowie der Schaumbereich ein Polyurethanmaterial umfassen, das erhalten wird, indem eine Zusammensetzung umfassend eine wässrige, anionisch hydrophilierte Polyurethandispersion (I) aufgeschäumt und getrocknet wird. Der geformte Artikel liegt folglich in Hinsicht auf den Schaumbereich und den komprimierten Bereich einstückig vor.

In einer Ausführungsform des geformten Artikels weist der komprimierte Bereich eine Dichte von ≥ 1 g/cm³ bis ≤ 1,8 g/cm³ auf. Die Dichte kann auch in einem Bereich von ≥ 1,2 g/cm³ bis ≤ 1,6 g/cm³ oder von ≥ 1,4 g/cm³ bis ≤ 1,5 g/cm³ liegen.

In einer weiteren Ausführungsform des geformten Artikels weist der komprimierte Bereich eine solche Transparenz aufweist, dass die Intensität von sichtbarem Licht nach dem Passieren des komprimierten Bereichs ≥ 50% bis ≤ 95% der ursprünglichen Intensität beträgt. Wie bereits ausgeführt, lässt sich hierdurch eine optische Kontrolle des abgedeckten Bereichs durchführen. Es ist auch möglich, dass die Intensität ≥ 60% bis ≤ 90% oder ≥ 70% bis ≤ 80% der ursprünglichen Intensität beträgt. Sichtbares Licht bezeichnet hierbei Licht mit einer Wellenlänge zwischen 380 nm und 780 nm. Zur Bestimmung der Transparenz kann beispielsweise weißes Licht herangezogen werden.

Gegenstand der Erfindung ist weiterhin die Verwendung eines geformten Artikels gemäß der vorliegenden Erfindung als Sportartikel, Textilartikel, Kosmetikartikel oder Wundauflage. Bevorzugt ist die Verwendung als Wundauflage. Vorteilhafterweise kann die Wundauflage so geformt sein, dass sie auf Körperteile aufgelegt werden kann. Ein Beispiel für ein Körperteil ist die Ferse, die Stirn, das Kinn, der Hals, der Beckenkamm oder das Gesäß. Weiterhin kann das Körperteil beispielsweise ein Gelenk sein. Hinsichtlich ihrer Größe ist die Wundauflage dem aufzunehmenden Körperteil wie der Ferse oder einem Gelenk angepasst, also beispielsweise einem Fingergelenk, einem Ellenbogengelenk, einem Kniegelenk oder einem Fußgelenk.

Die vorliegende Erfindung wird anhand der nachfolgenden Zeichnungen erläutert. Es zeigen:
FIG 1 eine perspektivische Ansicht eines erfindungsgemäß hergestellten geformten Artikels
FIG 2 eine Querschnittsansicht eines erfindungsgemäß hergestellten geformten Artikels

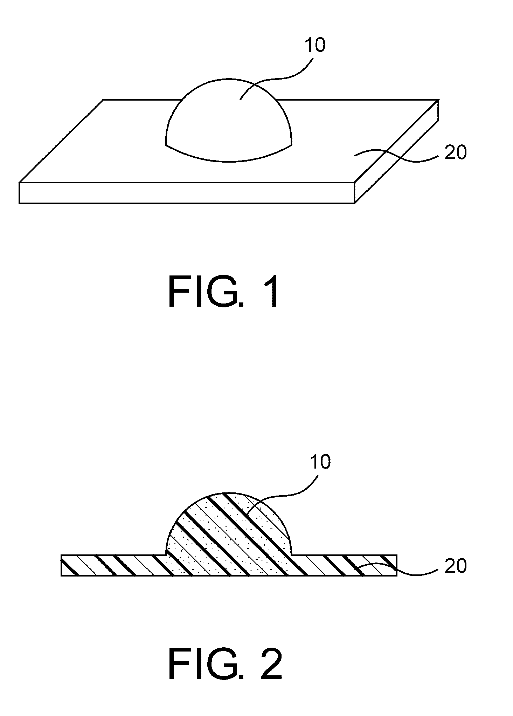

FIG 1 zeigt eine perspektivische Ansicht eines erfindungsgemäß hergestellten geformten Artikels. Der Schaumbereich 10 befindet sich in der Mitte des Artikels. Um den Schaumbereich herum liegt der komprimierte Bereich 20. Der Artikel wird hergestellt, indem ein Stück des erfindungsgemäß einzusetzenden Schaums mit den Abmessungen des fertigen Artikels in einer Form komprimiert und erwärmt wird. Die Unterseite der Form ist eben und weist keine Aussparungen auf. Die Oberseite der Form ist ebenfalls eben, weist jedoch in der Mitte eine kreisförmige Aussparung auf. Wird das Schaumstück nun dazwischen eingelegt und komprimiert, so führt die kreisförmige Aussparung dazu, dass der Schaumbereich 10 in Form einer Halbkugel erhalten bleibt, während der angrenzende Bereich zum komprimierten Bereich 20 umgeformt wird.

FIG 2 zeigt eine Querschnittsansicht eines erfindungsgemäß hergestellten geformten Artikels. Der Artikel ist derselbe Artikel wie in FIG 1. Man erkennt, wie durch die Aussparung in der Oberseite der Form der Schaumbereich 10 als Schaum erhalten blieb, während der angrenzende Bereich 20 komprimiert wurde. Die Unterseite des Artikels weist keine Lücken auf, sondern nur komprimierte Bereiche 20 oder Schaumbereiche 10.

### Ausführungsbeispiel

Ein wie vorstehend beschrieben erhältlicher Polyurethanschaum mit einem Raumgewicht von 180 kg/m³, entsprechend 0,18 g/cm³, und einer Dicke von 3,2 mm wurde bei einer Temperatur von 160 °C und einem Druck von 100 bar für eine Dauer zwischen 60 und 80 Sekunden auf eine Dicke von 0,4 mm, also 12,5% der ursprünglichen Dicke, verformt. Man erhielt einen kompakten Film.

## Patentansprüche

1. Verfahren zur Herstellung von geformten Artikeln,
wobei auf eine Schaumschicht in einem ersten Bereich bei einer Temperatur von ≥ 0 °C bis ≤ 200 °C ein Druck von ≥ 0 bar bis ≤ 150 bar einwirkt und während der Druckeinwirkung die Schaumschicht in dem ersten Bereich auf > 12,5% bis ≤ 100% ihres ursprünglichen Volumens komprimiert wird;
wobei auf eine Schaumschicht in einem zweiten Bereich bei einer Temperatur von ≥ 100 °C bis ≤ 200 °C ein Druck von ≥ 50 bar bis ≤ 150 bar einwirkt und während der Druckeinwirkung die Schaumschicht in dem zweiten Bereich auf > 0% bis ≤ 12,5% ihres ursprünglichen Volumens komprimiert wird; und
wobei die Schaumschicht in dem ersten Bereich und dem zweiten Bereich einen Polyurethanschaum umfasst, der erhalten wird, indem eine Zusammensetzung umfassend eine wässrige, anionisch hydrophilierte Polyurethandispersion (I) aufgeschäumt und getrocknet wird.

2. Verfahren gemäß Anspruch 1, wobei das Verformen des zweiten Bereichs für eine Dauer von ≥ 45 Sekunden bis ≤ 90 Sekunden durchgeführt wird.

3. Verfahren gemäß Anspruch 1, wobei die Zusammensetzung, aus der der Polyurethanschaum der Schaumschicht erhalten wird, weiterhin Zusatzstoffe umfasst, die ausgewählt sind aus der Gruppe umfassend Fettsäureamide, Sulfosuccinamide, Kohlenwasserstoffsulfonate, Kohlenwasserstoffsulfate, Fettsäuresalze, Alkylpolyglycoside und/oder Ethylenoxid/Propylenoxid-Blockcopolymere.

4. Verfahren gemäß Anspruch 3, wobei die Ethylenoxid/Propylenoxid-Blockcopolymere eine Struktur gemäß der allgemeinen Formel (1) aufweisen: 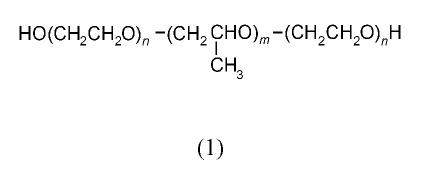 wobei der Wert für n in einem Bereich von ≥ 2 bis ≤ 200 liegt und der Wert für m in einem Bereich von ≥ 10 bis ≤ 60 liegt.

5. Verfahren gemäß Anspruch 1, wobei die wässrige, anionisch hydrophilierte Polyurethandispersion (I) erhältlich ist, indem
A) isocyanatfunktionelle Prepolymere bereitgestellt werden, welche erhältlich sind aus einer Reaktionsmischung umfassend
A1) organische Polyisocyanate und
A2) polymere Polyole mit zahlenmittleren Molekulargewichten von ≥ 400 g/mol bis ≤ 8000 g/mol und OH-Funktionalitäten von ≥ 1,5 bis ≤ 6
und wobei anschließend
B) die freien NCO-Gruppen der Prepolymere ganz oder teilweise mit
B1) isocyanatreaktiven anionischen oder potentiell anionischen Hydrophilierungsmitteln
unter Kettenverlängerung umgesetzt werden und die Prepolymere vor, während oder nach Schritt B) in Wasser dispergiert werden, wobei weiterhin in der Reaktionsmischung vorliegende potentiell ionische Gruppen durch teilweise oder vollständige Umsetzung mit einem Neutralisationsmittel in die ionische Form überführt werden.

6. Verfahren gemäß Anspruch 5, wobei die Reaktionsmischung in Schritt A) weiterhin umfasst:
A3) hydroxyfunktionelle Verbindungen mit Molekulargewichten von ≥ 62 g/mol bis ≤ 399 g/mol

7. Verfahren gemäß Anspruch 5, wobei die Reaktionsmischung in Schritt A) weiterhin umfasst:
A4) isocyanatreaktive, anionische oder potentiell anionische und gegebenenfalls nichtionische Hydrophilierungsmittel

8. Verfahren gemäß Anspruch 5, wobei in Schritt B) die freien NCO-Gruppen der Prepolymere weiterhin ganz oder teilweise umgesetzt werden mit
B2) aminofunktionelle Verbindungen mit Molekulargewichten von ≥ 32 g/mol bis ≤ 400 g/mol

9. Verfahren gemäß Anspruch 5, wobei bei der Herstellung der wässrigen, anionisch hydrophilierten Polyurethan-Dispersionen (I) die Komponente A1 ausgewählt ist aus der Gruppe umfassend 1,6-Hexamethylendiisocyanat, Isophorondiisocyanat und/oder die isomeren Bis-(4,4'-isocyanatocyclohexyl)methane und wobei weiterhin die Komponente A2) eine Mischung aus Polycarbonatpolyolen und Polytetramethylenglykolpolyolen umfasst, wobei der Anteil der Summe der Polycarbonatpolyole und der Polytetramethylenglykolpolyetherpolyole an der Komponente A2) ≥ 70 Gewichts-% bis ≤ 100 Gewichts-% beträgt.

10. Geformter Artikel, erhältlich durch ein Verfahren gemäß Anspruch 1, mit einem Schaumbereich (10) und einem komprimierten Bereich (20), wobei der komprimierte Bereich (20) aus der Kompression einer Schaumschicht erhalten wird und wobei diese Schaumschicht sowie der Schaumbereich (10) ein Polyurethanmaterial umfassen, das erhalten wird, indem eine Zusammensetzung umfassend eine wässrige, anionisch hydrophilierte Polyurethandispersion (I) aufgeschäumt und getrocknet wird.

11. Geformter Artikel gemäß Anspruch 10, wobei der komprimierte Bereich (20) eine Dichte von ≥ 1 g/cm³ bis ≤ 1,8 g/cm³ aufweist.

12. Geformter Artikel gemäß Anspruch 10, wobei der komprimierte Bereich (20) eine solche Transparenz aufweist, dass die Intensität von sichtbarem Licht nach dem Passieren des komprimierten Bereichs (20) ≥ 50% bis ≤ 95% der ursprünglichen Intensität beträgt.

13. Verwendung eines geformten Artikels gemäß Anspruch 10 als Sportartikel, Textilartikel, Kosmetikartikel oder Wundauflage.
